Europäisches Patentamt

(19) European Patent Office

Office européen des brevets

(11) Numéro de publication : **0 248 867 B1**

# (12) FASCICULE DE BREVET EUROPEEN

(45) Date de publication du fascicule du brevet :
27.02.91 Bulletin 91/09

(51) Int. Cl.⁵ : **A61B 5/07**, G01C 22/02

(21) Numéro de dépôt : 87900161.8

(22) Date de dépôt : 08.12.86

(86) Numéro de dépôt international :
**PCT/FR86/00424**

(87) Numéro de publication internationale :
**WO 87/03465 18.06.87 Gazette 87/13**

(54) **MODULE INGERABLE D'EXPLORATION FONCTIONELLE DU TUBE DIGESTIF.**

(30) Priorité : 09.12.85 FR 8518290

(43) Date de publication de la demande :
16.12.87 Bulletin 87/51

(45) Mention de la délivrance du brevet :
27.02.91 Bulletin 91/09

(84) Etats contractants désignés :
**AT BE CH DE FR GB IT LI LU NL SE**

(56) Documents cités :
**FR-A- 1 248 684**
**US-A- 3 350 944**
**US-A- 3 485 235**

(56) Documents cités :
**US-A- 3 769 711**
**US-A- 3 847 157**
**Electronics, vol. 49, no. 10, 13 May 1976, (N.Y., US), "Telemeter samples intestinal liquids", pages 3E, 4E.**

(73) Titulaire : **LAMBERT, Alain**
**1, rue des Mésanges**
**F-67370 Behlenheim (FR)**

(72) Inventeur : **LAMBERT, Alain**
**1, rue des Mésanges**
**F-67370 Behlenheim (FR)**

(74) Mandataire : **Nuss, Pierre**
**10, rue Jacques Kablé**
**F-67000 Strasbourg (FR)**

## Description

La présente invention concerne le domaine des accessoires de médecine pour l'exploration de certaines organes, en particulier du tube digestif, et a pour objet un module ingérable d'exploration fonctionnelle du tube digestif.

Actuellement, l'exploration fonctionnelle du tube digestif est généralement réalisée au moyen d'une sonde introduite dans l'intestin par la bouche du patient à travers l'estomac et le pylore ou par l'anus. Ce mode d'exploration connu occasionne, cependant, une gêne importante pour le patient pendant toute la durée des investigations, et, en outre, il ne permet pas d'assurer l'exploration de toute la longueur de l'intestin. Enfin, ce mode d'exploration connu ne permet pas de situer correctement la position de l'extrémité de la sonde à un moment donné. Un module ingérable d'exploration fonctionnelle du tube digestif se présentant sous forme d'une capsule allongée de faible diamètre et comportant une source d'énergie électrique est connue du "Electronics, vol. 9, no. 10, 1976, Telemeter samples intestinal liquids".

La présente invention a pour but de pallier ces inconvénients.

Elle a, en effet pour objet un module ingérable d'exploration fonctionnelle du tube digestif se présentant sous forme d'une capsule allongée de faible diamètre et comportant une source d'énergie électrique, caractérisé en ce qu'il comporte un moyen de mesure de déplacement délivrant des impulsions de déplacement à un circuit émetteur électronique de transmission de données à un récepteur extérieur correspondant, et la source d'énergie électrique assurant l'alimentation du circuit.

L'invention sera mieux comprise grâce à la description ci-après, qui se rapporte à des modes de réalisation préférés, donnés à titre d'exemples non limitatifs, et expliqués avec référence au dessin schématique annexé, dans lequel :

la figure 1 est une vue en élévation latérale et en coupe d'un module conforme à une réalisation préférable de l'invention,

la figure 2 est une vue en plan et partiellement en coupe suivant la figure 1 ;

la figure 3 est une vue analogue à celle de la figure 2 à plus grande échelle d'une variante de réalisation de l'invention, et

la figure 4 est une vue analogue à celle de la figure 1 d'une troisième variante de réalisation de l'invention.

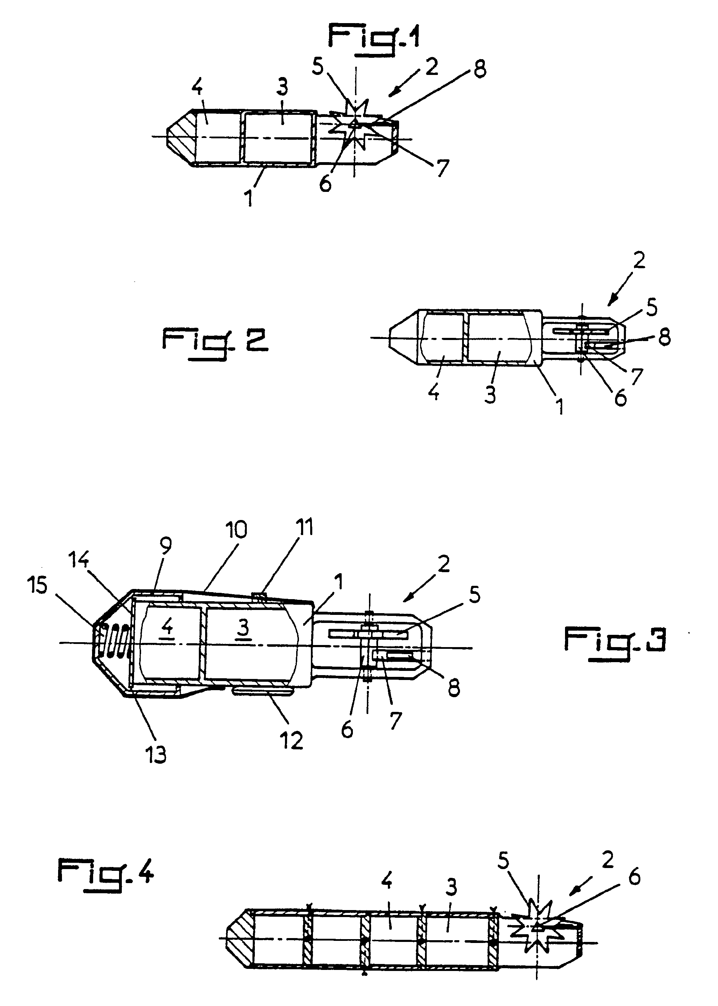

Conformément à l'invention, et comme le montrent plus particulièrement, à titre d'exemple, les figures 1 et 2 des dessins annexés, le module ingérable d'exploration fonctionnelle du tube digestif se présente sous forme d'une capsule allongée 1 de faible diamètre qui comporte un moyen 2 de mesure de déplacement délivrant des impulsions de déplacement à un circuit émetteur électronique 3 qui transmet les données à un récepteur extérieur correspondant, ce circuit 3 étant alimenté par une source d'énergie électrique 4.

Le moyen 2 de mesure de déplacement est avantageusement constitué par une roue dentée 5 montée à rotation libre dans le corps de la capsule 1, en saillie, d'un côté, sur le contour du corps de la capsule 1, et pourvue d'un axe 6, de section polygonale (figure 2), de préférence de section triangulaire, cette roue 5 étant montée dans la capsule 1 à poste fixe ou escamotable dans la capsule 1 contre l'action d'un ressort, par une lamelle flexible 7 encastrée à une extrémité dans le corps de la capsule 1 et s'appuyant à son autre extrémité sur l'axe 6, le plan de cette lamelle 7 passant par l'axe de rotation de la roue 5, et par une jauge de contrainte 8 collée sur la lamelle 7.

Le circuit émetteur électronique 3 de transmission de données à un récepteur extérieur correspondant est un circuit électronique miniaturisé de type connu, qui est relié, d'une part, à la jauge de contrainte 8 et, d'autre part, à la source d'énergie électrique 4 qui est avantageusement constituée par des piles de type connu.

Suivant le sens de rotation de la roue dentée 5, l'axe 6 soulève ou abaisse l'extrémité de la lamelle 7, et ce à raison de trois fois par tour de roue, dans le cas de l'axe suivant la figure 1. Les flexions ainsi produites entraînent une diminution ou une augmentation de la résistance de la jauge de contrainte 8 traversée par un léger courant électrique, créant une variation de tension relevée et transmise par le circuit émetteur 3 au récepteur extérieur. Ce dernier, qui est avantageusement raccordé, de manière connue, à un enregistreur graphique, permet alors d'effectuer un tracé représentant les flexions de la lamelle 7, le décriptage de ces flexions permettant de déduire le nombre de tours de la roue 5 et son sens de rotation, la distance parcourue par le module et le rythme ou la variation du rythme de progression.

Après mise sous tension du circuit émetteur 3 au moyen de la source 4, le module est déposé dans l'estomac du patient au moyen d'une sonde ou avalé, et il franchit le pylore au bout d'un certain laps de temps. Ce franchissement du pylore est détectable par surveillance des rotations de la roue dentée 5, qui, après avoir été désordonnées, deviennent cohérentes et indiquent une progression du module dans la lumière intestinale. A titre d'exemple, l'enregistrement des signaux émis par le circuit 3 peut être effectué pendant environ dix heures, et le module est récupéré dans les selles quelques jours après. Ce module est réutilisable après nettoyage total et remplacement de la source 4.

L'exploration au moyen du module suivant les figures 1 et 2 permet de connaître, d'une part, les modalités de la progression dudit module dans le tube digestif dans les cas normaux et pathologiques et ainsi de mieux cerner les problèmes liés à la motricité de l'intestin, d'autre part, la longueur de l'intestin, un intestin court pouvant être responsable d'un certain nombre de désordres physiologiques, et enfin l'effet de drogues sur la motricité intestinale.

La figure 3 représente une variante de réalisation de l'invention, dans laquelle, le module est muni, en outre, d'une capsule de succion 9 fixée de manière détachable sur l'une de ses extrémités au moyen d'un fil 10 solidarisé à ses extrémités avec le corps de la capsule 1 l'une des extrémités du fil 10 traversant, en outre, un dispositif de rupture du fil 10, mécanique ou chimique ou un microfour 11 relié à la source 4 et actionné par un contacteur magnétique 12 dont la commande est réalisée au moyen d'un champ magnétique extérieur.

La capsule de succion 9 est sous forme d'un corps cylindrique ou en ogive muni d'une enveloppe mobile 9 et d'un fond fixe 14, l'enveloppe 9 étant déplaçable sous la charge d'un ressort 15 monté dans la capsule ou enveloppe 9, le fond fixe 14 présentant un jeu annulaire 13 avec l'enveloppe de la capsule 9 assurant le remplissage de cette dernière par succion, le diamètre intérieur de cette capsule 9 étant tel qu'elle s'emmanche sur la capsule 1 par compression du ressort 15 au moyen du fond 14 pour sa fixation au moyen du fil 10. Ainsi, après ingestion, lorsque le module a atteint la zone désirée, il suffit de créer un champ magnétique pour fermer le contacteur 12 qui met le microfour 11 sous tension, ce dernier provoquant la rupture du fil 10 par fusion. La capsule 9 est alors larguée automatiquement sous l'effet de la poussée du ressort 15 sur le fond fixe 14, cette poussée provoquant simultanément un effet de succion à travers le jeu annulaire 13 du fond fixe 14, l'arrivée en fin de course de la capsule 9 par rapport au fond 14 fermant hermétiquement ladite capsule 9 et empêchant toute fuite du liquide recueilli. Ce dernier peut alors être soumis à des études enzymatiques ou à d'autres analyses.

Conformément à une variante de réalisation de l'invention, la capsule 9 est munie d'un orifice de remplissage pourvu d'une soupape anti-retour, ou d'un dispositif de colmatage dudit orifice, le fond fixe étant guidé à étanchéité dans la capsule 9.

Selon une autre caractéristique de l'invention, non représentée au dessin annexé, le module peut être pourvu, dans la capsule 1 d'une ou de plusieurs soutes munies chacune d'une vanne dont la commande d'ouverture et de fermeture est effectuée par télécommande, ou mécaniquement au moyen de circuits à temporisation indépendants et préréglés, ou au moyen de pistons ouvrant et fermant des orifices de remplissage ou d'évacuation. Un tel mode de réalisation permet également de réaliser des prélèvements à des fins d'analyses, mais également de déposer, le cas échéant, une drogue pharmaceutique ou un autre médicament en un point bien précis du tube digestif.

Conformément à une autre caractéristique de l'invention, le module peut être pourvu, en outre, de capteurs de température, de pH, de pression, et d'électrodes ou autres détecteurs reliés électriquement au circuit 3. De tels capteurs permettent l'enregistrement en continu d'un certain nombre de variables aux différents niveaux du tube digestif, en particulier la pression intraluminale, l'activité électrique des muscles intestinaux, les contractions circulaires, ou tout autre paramètre.

Selon une autre variante de réalisation de l'invention, et comme le montre la figure 4, la capsule 1 peut avantageusement être constituée par des sous-ensembles assemblés de manière séparable à leurs extrémités, chaque sous-ensemble renfermant une partie des éléments constitutifs d'un module à savoir le moyen 2, le circuit 3 la source d'énergie 4, et autres, la liaison électrique entre les sous-ensembles étant réalisée au moyen de plots de contact.

Conformément à une caractéristique d'une réalisation préférable de l'invention, la liaison séparable entre les sous-ensembles formant la capsule 1 est réalisée au moyen de fil résorbable maintenant les sous-ensembles entre eux. Ainsi, il est possible de réaliser un module ingérable dont l'agressivité pour le tube digestif, après enregistrement des paramètres, est amoindrie, en particulier le risque de blocage.

Le module conforme à l'invention, permet de fournir simultanément plusieurs informations sur le tube digestif, à savoir, outre la longueur de l'intestin grêle, la vitesse de progression d'un solide et les modalités de progression de celui-ci. Ainsi, il a pu être mis en évidence, de façon indiscutable des mouvements anti-péristaltiques intestinaux, qui sont surtout fréquents au niveau du jéjunum et de l'iléon, ceci en particulier, grâce à la précision du sens de rotation de la roue 5 du module.

Ce dernier a permis, en outre de démontrer que l'administration de certains tranquillisants peut ralentir de manière très importante le transit grêle, tandis que d'autres substances entraînent une progression rapide du module avec des phases d'accélération très importantes. L'invention est donc particulièrement intéressante dans le domaine pharmaceutique pour les essais d'efficacité de médicaments.

En outre, l'utilisation de la capsule de succion 9 permettant le prélèvement de suc digestif à des niveaux précis, par exemple au jéjunum ou à l'iléon, permet l'étude de l'action de ces sucs sur un nutriment déterminé.

Enfin, le module selon l'invention permet d'administrer des médicaments d'une façon plus scientifique, c'est-à-dire en tenant compte à la fois des rythmes biologiques et des interférences sur le plan digestif d'un certain nombre de facteurs, tels que le pH ou les phénomènes enzymatiques. Ainsi, un médicament pourra être lâché à un niveau bien déterminé de l'intestin grêle où son action sera la plus favorable.

Bien entendu, l'invention n'est pas limitée aux modes de réalisation décrits et représentés aux dessins annexés. Des modifications restent possibles, notamment du point de vue de la constitution des divers éléments, ou par substitution d'équivalents techniques, sans sortir pour autant du domaine de protection de l'invention.

**Revendications**

1. Module ingérable d'exploration fonctionnelle du tube digestif se présentant sous forme d'une capsule allongée (1) de faible diamètre et comportant une source d'énergie électrique (4), caractérisé en ce qu'il comporte un moyen (2) de mesure de déplacement délivrant des impulsions de déplacement à un circuit émetteur électronique (3) de transmission de données à un récepteur extérieur correspondant, et la source d'énergie électrique (4) assurant l'alimentation du circuit (3).

2. Module suivant la revendication 1, caractérisé en ce que le moyen (2) de mesure de déplacement est constitué par une roue dentée (5) montée à rotation libre dans le corps de la capsule (1), en saillie, d'un côté, sur le contour du corps de la capsule (1), et pourvue d'un axe (6), de section polygonale, de préférence de section triangulaire, cette roue (5) étant montée dans la capsule (1) à poste fixe ou escamotable dans la capsule (1) contre l'action d'un ressort ; par une lamelle flexible (7) encastrée à une extrémité dans le corps de la capsule (1) et s'appuyant à son autre extrémité sur l'axe (6), le plan de cette lamelle (7) passant par l'axe de rotation de la roue (5) ; et par une jauge de contrainte (8) collée sur la lamelle (7).

3. Module, suivant l'une quelconque des revendications 1 et 2, caractérisé en ce que le circuit émetteur électrique (3) de transmission de données à un récepteur extérieur correspondant est un circuit électronique miniaturisé de type connu, qui est relié, d'une part, à la jauge de contrainte (8), et, d'autre part à la source d'énergie électrique (4), qui est avantageusement constituée par des pièces de type connu.

4. Module suivant l'une quelconque des revendications 1 à 3, caractérisé en ce qu'il est muni, en outre, d'une capsule de succion (9) fixée de manière détachable sur l'une de ses extrémités au moyen d'un fil (10) solidarisé à ses extrémités avec le corps de la capsule (1) l'une des extrémités du fil (10) traversant, en outre, un dispositif de rupture du fil (10), mécanique ou chimique, ou un microfour (11) relié à la source (4), et actionné par un contacteur magnétique (12), dont la commande est réalisée au moyen d'un champ magnétique extérieur.

5. Module, suivant la revendication 4, caractérisé en ce que la capsule de succion (9) est sous forme d'un corps cylindrique ou en ogive muni d'une enveloppe mobile (9) et d'un fond fixe (14), l'enveloppe (9) étant déplaçable sous la charge d'un ressort (15) monté dans la capsule ou enveloppe (9), le fond fixe (14) présentant un jeu annulaire (13) avec l'enveloppe de la capsule (9) assurant le remplissage de cette dernière par succion, le diamètre intérieur de cette capsule (9) étant tel qu'elle s'emmanche sur la capsule (1) par compression du ressort (15) au moyen du fond (14) pour sa fixation au moyen de fil (10).

6. Module, suivant l'une quelconque des revendications 4 et 5, caractérisé en ce que la capsule (9) est munie d'un orifice de remplissage pourvu d'une soupape anti-retour, ou d'un dispositif de colmatage dudit orifice, le fond fixe étant guidé à étanchéité dans la capsule (9).

7. Module, suivant la revendication 1, caractérisé en ce qu'il est pourvu dans la capsule (1) d'une ou de plusieurs soutes munies chacune d'une vanne, dont la commande d'ouverture et de fermeture est effectuée par télécommande, ou mécaniquement au moyen de circuits à temporisation indépendants et préréglés, ou au moyen de pistons ouvrant et fermant des orifices de remplissage ou d'évacuation.

8. Module, suivant l'une quelconque des revendications 1 à 7, caractérisé en ce qu'il est pourvu, en outre, de capteurs de température, de pH, de pression et d'électrodes, ou autres détecteurs reliés électriquement au circuit (3).

9. Module, suivant la revendication 1, caractérisé en ce que la capsule (1) est avantageusement constituée par des sous-ensembles assemblés de manière séparable à leurs extrémités, chaque sous-ensemble renfermant une partie des éléments constitutifs d'un module, à savoir le moyen (2), le circuit (3), la source d'énergie (4) et autres, la liaison électrique entre les sous-ensembles étant réalisée au moyen de plots de contact.

10. Module suivant la revendication 9, caractérisé en ce que la liaison séparable entre les sous-ensembles formant la capsule (1), est réalisée au moyen de fil résorbable maintenant les sous-ensembles entre eux.

**Ansprüche**

1. Verschluckbare Einheit zur funktionellen Untersuchung einer Speiseröhre, die in Form einer länglichen Kapsel (1) mit kleinem Durchmesser ausgelegt ist und eine elektrische Energiequelle (4) hat, dadurch **gekennzeichnet**, daß sie eine Einrichtung (2) zur Verschiebungsmessung aufweist, die Verschiebungsimpulse an eine elektronische Emitterschaltung (3) zur Informationsübertragung an einen zugeordneten externen Empfänger liefert, und daß die elektrische Energiequelle (4) die Versorgung der Schaltung (3) sicherstellt.

2. Verschluckbare Einheit nach Anspruch 1, dadurch gekennzeichnet, daß die Einrichtung (2) zur Verschiebungsmessung von einem mit Zähnen versehenen Rad (5) gebildet wird, das frei drehbeweglich in dem Körper der Kapsel (1) mit einer Erhebung an einer Seite, die über den Körper der Kapsel (1) vorsteht, gelagert ist, und das eine Achse (6) mit einem polygonalen, vorzugsweise dreieckförmigen Querschnitt aufweist, daß das Rad (5) in der Kapsel (1) an einem festen oder einziehbaren Träger in der Kapsel (1) entgegen der Wirkung einer Feder gelagert ist, und daß eine flexible Lamelle (7) vorgesehen ist, die in ein Ende in den Körper der Kapsel (1) eingebaut ist und gegen das andere Ende auf der Achse (6) anliegt, wobei die Ebene dieser Lamelle (7) durch die Drehachse des Rads (5) geht, und daß ein Dehnungsmeßstreifen (8) auf die Lamelle (7) geklebt ist.

3. Verschluckbare Einheit nach einem der Ansprüche 1 und 2, dadurch gekennzeichnet, daß die elektrische Emitterschaltung (3) zur Informationsübertragung an einen zugeordneten externen Empfänger eine elektronische Schaltung in Miniaturbauweise an sich bekannter Art ist, die einerseits mit dem Dehnungsmeßstreifen (8) und andererseits mit der Energiequelle (4) verbunden ist, die vorzugsweise von an sich bekannten Teilen gebildet wird.

4. Verschluckbare Einheit nach einem der Ansprüche 1 bis 3, dadurch gekennzeichnet, daß sie ferner mit einer verschluckbaren Kapsel (9) versehen ist, die lösbar an einem der Enden mit Hilfe eines Fadens (10) angebracht ist, der an seinen anderen Enden fest mit dem Körper der Kapsel (1) verbunden ist, daß eines der Enden des Fadens (10) ferner durch eine mechanische oder chemische Durchtrenneinrichtung des Fadens (10) oder einen Mikroofen (11) geht, der mit der Quelle (4) verbunden ist und durch einen magnetischen Kontakt (12) gesteuert wird, der mit Hilfe eines externen Magnetfeldes ansteuerbar ist.

5. Verschluckbare Einheit nach Anspruch 4, dadurch gekennzeichnet, daß die verschluckbare Kapsel (9) in Form eines zylindrischen oder eines raketenförmigen Körpers ausgebildet ist, der mit einer beweglichen Hülle (9) und einem festen Boden (14) versehen ist, daß die Hülle (9) durch die Belastung einer Feder (15) verschiebbar ist, die in der Kapsel oder Hülle (9) vorgesehen ist, der feste Boden (14) einen Ringzwischenraum (13) in Verbindung mit der Hülle der Kapsel (9) bildet, wodurch ein Auffüllen derselben mittels Schlucken sichergestellt wird, und daß der Innendurchmesser der Kapsel (9) derart gewählt ist, daß er auf die Kapsel (1) durch die Kompression der Feder (15) im Zusammenwirken mit dem Boden (14) zur Fixierung mit Hilfe des Fadens (10) aufgepreßt ist.

6. Verschluckbare Einheit nach einem der Ansprüche 4 und 5, dadurch gekennzeichnet, daß die Kapsel (9) mit einer Füllöffnung versehen ist, die mit einem Rückschlagventil oder einer Verschlußeinrichtung für die Öffnung versehen ist, und daß der feste Boden dicht in der Kapsel (9) geführt ist.

7. Verschluckbare Einheit nach Anspruch 1, dadurch gekennzeichnet, daß in der Kapsel (1) ein oder mehrere Vorratsräume vorgesehen sind, die mit einem Schieber versehen sind, dessen Öffnungs- und Schließbewegung ferngesteuert oder mechanisch mit Hilfe von Verzögerungsschaltungen gesteuert werden, die unabhängig und voreinstellbar sind, oder mit Hilfe von Kolben gesteuert werden, welche die Füllöffnungen oder die Abzugsöffnungen öffnen und verschließen.

8. Verschluckbare Einheit nach einem der Ansprüche 1 bis 7, dadurch gekennzeichnet, daß sie ferner mit Sensoren für die Temperatur, für den pH-Wert, den Druck und die Elektroden oder weiteren Detektoren versehen ist, die mit der Schaltung (3) elektrisch verbunden sind.

9. Verschluckbare Einheit nach Anspruch 1, dadurch gekennzeichnet, daß die Kapsel (1) zweckmäßigerweise von Unterbaugruppen derart gebildet wird, daß diese an ihren Enden voneinander trennbar sind, und daß jede Unterbaugruppe einen Teil der Bauelemente einer Einheit, wie die Einrichtung (2) die Schaltung (3), die Energiequelle (4) und weitere aufnimmt, und daß die elektrische Verbindung unter den Unterbaugruppen mit Hilfe von Kontaktsätzen vorgesehen ist.

10. Verschluckbare Einheit nach Anspruch 9, dadurch gekennzeichnet, daß die zwischen den Unterbaugruppen, die die Kapsel (1) bilden, trennbare Verbindung in Form eines resorbierbaren Fadens ausgelegt ist, welcher die Unterbaugruppen untereinander zusammenhält.

## Claims

1. Ingestable module for the functional exploration of the digestive tract in the form of an elongate capsule (1) having a small diameter and comprising an electric power source (4), characterised in that it comprises a displacement measuring means (2) delivering displacement pulses to an electronic emitter circuit (3) for transmission of data to a corresponding external receiver, the electric power source (4) supplying the circuit (3).

2. Module according to claim 1, characterised in that the displacement measuring means (2) consists of a toothed wheel (5) mounted freely rotatably in the body of the capsule (1), projecting on one side on the contour of the body of the capsule (1) and provided with a shaft (6) of polygonal cross section, preferably of triangular cross section, this wheel (5) being mounted in the capsule (1) in a fixed or retractable position in the capsule (1) against the action of a spring ; of a flexible blade (7) fitted at one end in the body of the capsule (1) and resting at its other end on the shaft (6), the plane of this blade (7) passing through the axis of rotation of the wheel (5) ; and of a strain gauge (8) stuck on the blade (7).

3. Module according to any one of claims 1 and 2, characterised in that the electric emitter circuit (3) for transmission of data to a corresponding external receiver is a miniaturised electronic circuit of a known type which is connected, on the one hand, to the strain gauge (8) and, on the other hand, to the electric power source (4) which advantageously consists of parts of a known type.

4. Module according to any one of claims 1 to 3, characterised in that it is also equipped with a suction capsule (9) detachably fixed at one of its ends by means of a wire (10) attached at its ends to the body of the capsule (1), one of the ends of the wire (10) also traversing a mechanical or chemical wire (10) breaking device or a microfurnace (11) connected to the source (4) and actuated by a magnetic contact switch (12) which is controlled by means of an external magnetic field.

5. Module according to claim 4, characterised in that the suction capsule (9) is in the form of a cylindrical or ogive-shaped body equipped with a moving casing (9) and a fixed base (14), the casing (9) being displaceable under the load of a spring (15) mounted in the capsule or casing (9), the fixed base (14) having an annular play (13) with the casing of the capsule (9) allowing the capsule (9) to be filled by suction, the internal diameter of this capsule (9) being such that it fits over the capsule (1), due to compression of the spring (15) by means of the base (14), for the fixing thereof by means of wire (10).

6. Module according to any one of claims 4 and 5, characterised in that the capsule (9) is equipped with a filling orifice provided with a nonreturn valve or with a device for sealing said orifice, the fixed base being guided with a tight seal in the capsule (9).

7. Module according to claim 1, characterised in that it is provided, in the capsule (1), with one or more bays each equipped with a valve, the opening and closure of which is controlled remotely or mechanically by means of independent, preset time delay circuits or by means of pistons which open and close the filling or discharge orifices.

8. Module according to any one of claims 1 to 7, characterised in that it is also provided with temperature, pH and pressure sensors and with electrodes or other detectors electrically connected to the circuit (3).

9. Module according to claim 1, characterised in that the capsule (1) advantageously consists of sub-assemblies which are separably connected at their ends, each sub-assembly containing some of the constituent elements of a module, that is the means (2), the circuit (3), the power source (4) etc., the electrical connection between the sub-assemblies being produced by means of bonding pads.

10. Module according to claim 9, characterised in that the separable connection between the sub-assemblies forming the capsule (1) is produced by means of resorbable wire holding the sub-assemblies together.

Fig.1

Fig.2

Fig.3

Fig.4